# EUROPEAN PATENT SPECIFICATION

(11) **EP 2 422 845 B1**
(45) Date of publication and mention of the grant of the patent: **24.04.2013**
(21) Application number: 10173896.1
(22) Date of filing: 24.08.2010
(51) Int. Cl.: A61N 5/06

(54) **Lightguide phototherapy apparatus**
Lichtleiter-Phototherapievorrichtung
Appareil de photothérapie à guide d'ondes

(43) Date of publication of application: 29.02.2012
(73) Proprietor: Polyphotonix Limited, Sedgefield County, Durham TS21 3FG (GB)
(72) Inventor: Veres, Janos, Pittsford, New York 14534 (US); MIREMONT, Christophe Olivier, South Queensferry EH30 9HR (GB); HILL, Duncan John, Sedgefield County Durham TS21 3FG (GB)
(74) Representative: Harrison Goddard Foote

(56) References cited:
- WO-A1-2004/064922
- US-A- 4 777 937
- US-A1- 2010 179 469

## Description

The present invention relates to apparatus for radiation treatment devices of the eye. The invention relates particularly, but not exclusively, to radiation treatment apparatus for treating diabetic retinopathy.

### Background of the invention

Light treatment of the eye can be used for a variety of therapeutic purposes, including retinopathy, modifying biological rhythms, psychiatric disorders and depression. For example, diabetic retinopathy is a condition caused by diabetes in which damage to the retina occurs, which can lead to impairment of vision or blindness. The condition can be treated by preventing the complete dark adaptation of the eye by illuminating the eyes/eyelids during sleep. The required wavelength for this treatment corresponds to the scotopic sensitivity of the eye, approximately 480 to 510 nm. Ideally a comfortable mask would be required for light treatments on the eye, especially if the therapy is conducted during sleep.

Several examples of light therapy devices acting on the eye are known. Most of these utilize LEDs as light source. LEDs are substantially rigid and most configurations cause some degree of discomfort to the user, especially if the device is to be used while sleeping. In particular LED sources generate heat, causing discomfort. Some examples of LED based devices are below:
GB2410903A (Arden) describes a light emitting device for preventing retinal disease. The device is based on LED emitters.
US6,350,275 (Vreman) describes a radiation treatment device with LED emitters. The LEDs are integrated into the frames of eyeglasses.
US4,858,609 (Cole) describes a bright light mask with the external light source coupled via fibre optics. Such a solution requires a separate light source, which is cumbersome for the wearer. Alternatively, the document suggests miniature light bulbs embedded in the mask. In this implementation, heat filtering is proposed.
US4,396,259 (Miller) describes eyeglasses with a spectrum wheel. The device uses a lightbulb, making it cumbersome and bulky.
EP 0495988 (Masuda) describes light emitting glasses to induce sleep, utilizing light emitting diodes.
US5,486,880 (House) describes an apparatus for causing constriction of the pupil of an eye. The disclosure does not address the type of light source to be used in such devices.
US 4,938,582 (Leslie) describes a chromo therapy device that utilizes LEDs projecting light onto a screen in front of the eyes. The proposed device is essentially a pair of glasses which are bulky in construction.
US 6,319,273 (Chen) describes an illuminating device for treating eye disease. The invention utilizes LEDs and proposes the use of a focusing lens.
US2008/0269849 (Lewis) describes pulsed phototherapy devices, including eyemasks employing LEDs or lasers. The invention does not offer efficient solutions for heat management.

It is clear that LED and incandescent based devices have disadvantages in terms of either having rigid components or dissipation of heat generated by spot sources. The use of flat electroluminescent films is one possibility to make the device thin and deal with heat management. For example, some of the prior art suggests the use of so called AC electroluminescent (ACEL) films or wires. While the form factor of such films is ideal, they operate at high voltages and therefore are not preferred.

US 2005/0278003 (Feldman) describes wearable devices to deliver light to the retina. The disclosure proposes the use of LED, electroluminescent wire or electroluminescent flat panels. The disclosure does not offer power efficient solutions.

US4,777,937 (Rush) describes a mood altering facial mask with an electroluminescent strip that provides stimulus of a particular colour. The electroluminescent strip operates at high voltages and its power efficiency is not ideal.

US 2010/0179469 (Hammond) suggests the use of OLED based phototherapy systems for the stimulation of the retina. The disclosure recognizes the need for heat management, and suggests heat sinks or other dedicated cooling such as cooling fans to solve the problem. The disclosure does not offer means to reduce the heat emitted per area.

There is a need for improved eye masks for phototherapy that are comfortable to wear, dissipate little heat, and operate efficiently, with minimal power requirements.

Preferred embodiments of the present invention seek to overcome one or more of the disadvantages discussed above of known arrangements.

### Summary of the invention

According to an aspect of the present invention, there is provided an apparatus for directing electromagnetic radiation into a respective pupil of at least one eye of a user, the apparatus comprising:
first radiation emitting means comprising at least one radiation emitting layer adapted to emit electromagnetic radiation;
second radiation emitting means adapted to direct electromagnetic radiation into at least one eye and/or onto at least one eyelid of a user; and
radiation guide means adapted to receive radiation emitted by said first radiation emitting means and to direct at least part of said radiation to said second radiation emitting means, wherein a total surface area of said first emitting radiation means from which radiation is emitted is larger than a total cross sectional area of a beam of radiation entering the or each pupil of the user in use, in a direction transverse to an optical axis of said beam.

By providing first and second radiation emitting means such that a total surface area of said first radiation emitting means from which radiation is emitted is larger than a total cross sectional area of a beam of radiation entering the or each pupil of the user in use, in a direction transverse to an optical axis of said beam, this provides the advantage of enabling radiation directed into one or more eyes of the user to be generated over a larger area than in the prior art. This in turn provides the advantage of enabling the necessary therapeutic radiation doses to be generated at lower intensity than in the prior art, which reduces the heat generated per unit area, thus minimising discomfort to the user. In addition, under certain circumstances, this enables the radiation to be generated more efficiently.

At least one said radiation emitting layer may be adapted to emit radiation in response to application of electrical power.

At least one said radiation emitting layer may be adapted to emit radiation in response to irradiation of said layer.

By suitable choice of materials, this provides the advantage of enabling a more comfortable and/or a disposable apparatus to be provided, for example an apparatus which emits radiation over an extended period in response to initial excitation.

At least one said radiation emitting layer may comprise at least one organic light emitting diode (OLED).

The radiation guide means may comprise a radiation transmitting portion including at least one first layer having a respective first refractive index and at least one second layer having a respective second refractive index, wherein said first refractive index of at least one said first layer is higher than the respective second refractive index of at least one second layer in contact with said first layer.

This provides the advantage of minimising radiation loss out of said first layer by causing total internal reflection of the radiation.

The radiation guide means may include at least one luminescent material.

This provides the advantage of enabling the predominant wavelength of radiation entering an eye of the user to be selected.

The degree of luminescence of at least one said luminescent material may vary with time. For example, in a case in which the luminance of a material in said first radiation means decreases with time, the radiation guide means may include at least one photochromic layer material having a transparency which increases with time, so that decay in the photochromic layer compensates decay in the material of the first radiation means.

The luminescent material may be concentrated in a region adjacent said second radiation emitting means. This provides the advantage of minimising the amount of luminescent material needed to be used.

The radiation guide means may further comprise at least one reflective layer.

This provides the advantage of further minimising leakage of radiation from the radiation guide means, as a result of which the intensity of radiation directed to the eye of the user can be maximized, which in turn enables the intensity of the radiation source to be minimized, further reducing the degree of discomfort to the user.

The radiation guide means may comprise a radiation transmitting portion having tapered thickness adjacent at least one edge portion thereof.

This provides the advantage of enabling radiation leakage from the radiation guide means to be minimised under certain circumstances. For example, the radiation leakage could be minimised as a result of increasing angle of reflection from a second layer having lower refractive index adjacent to a first layer having higher refractive index, thus further contributing to total internal reflection.

The radiation guide means may comprise a radiation transmitting portion, wherein the radiation transmitting portion is rounded adjacent at least one edge portion thereof.

This provides the advantage of further minimising radiation leakage.

The second radiation emitting means may be adapted to conform to a shape of at least one eyelid of the user.

The second radiation emitting means may include at least one coolable material.

The second radiation emitting means may include at least one material having a transmissivity to radiation varying with radiation intensity.

This provides the advantage of enabling the intensity of radiation directed to an eye of the user to be regulated.

At least one said radiation emitting layer may be adapted to be moved between a respective first position, in which said layer is adapted to direct radiation into said radiation guide means, and a respective second position, in which said layer is adapted to be exposed to exciting radiation.

This provides the advantage of enabling a more compact apparatus to be provided.

At least one said radiation emitting layer may be adapted to be moved between respective first and second positions thereof by folding.

Preferred embodiments of the invention will now be described, by way of example only and not in any limitative sense, with reference to the accompanying drawings, in which:
Figure 1 is a perspective view of an eye mask of a first embodiment of the present invention;
Figure 2 is a perspective view of an eye mask of a second embodiment of the present invention;
Figure 3 is a perspective view of an eye mask of a third embodiment of the present invention;
Figure 4 is a side cross-sectional view of the eye mask of Figure 1;
Figure 5 is a side cross-sectional view illustrating a process of optically charging the eye mask of Figure 4;
Figure 6 is a side cross-sectional view of an eye mask of a fourth embodiment of the present invention;
Figure 7 is a side cross-sectional view of an eye mask of a fifth embodiment of the present invention;
Figures 8(i) to 8(iii) show a side cross-sectional view of an eye mask of a sixth embodiment of the present invention;
Figure 9 is a perspective view of the eye mask of Figures 8(i) to 8 (iii);
Figure 10 is a side cross-sectional view of an eye mask of a seventh embodiment of the present invention;
Figure 11 is a side cross-sectional view of an eye mask of a eighth embodiment of the present invention;
Figure 12 is a side cross-sectional view of an eye mask of a ninth embodiment of the present invention; and
Figure 13 is a side cross-sectional view of an eye mask of a tenth embodiment of the present invention.

The present invention is based on the discovery that heat dissipation in light sources for eye masks can be effectively solved by using area light sources and by extending their size so that they are significantly larger than the eye. The light intensity generated per unit area can therefore be reduced and thus the associated heat dissipation per unit area is also reduced. To compensate for the reduced luminance, the total light output is effectively coupled into a lightguide, collected and emitted into the eye or onto the eyelids.

### Detailed description

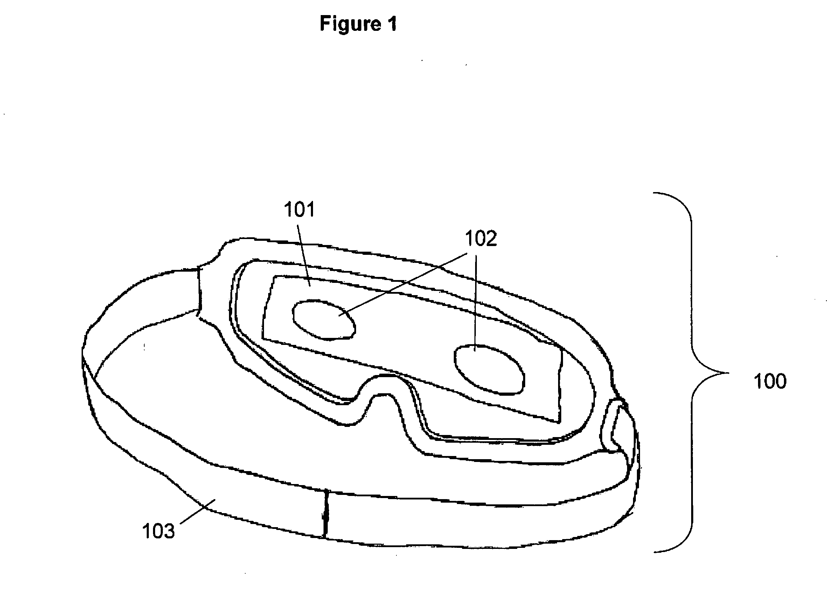

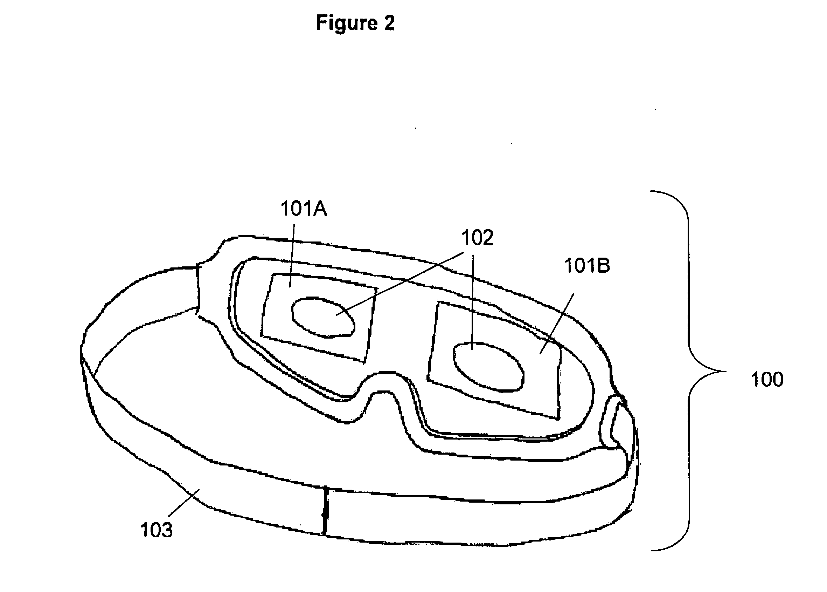

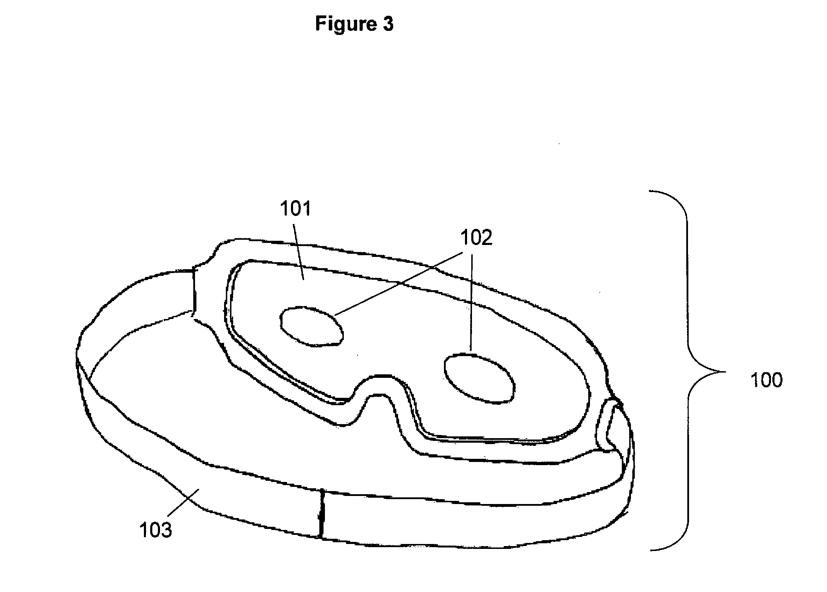

An eyemask of a first embodiment of the present invention is shown in Figure 1, where 100 is the eyemask itself, and 101 illustrates an area light emitter combined with a lightguide and which is substantially larger than light exit windows 102 designed to illuminate the eyes. The mask can be attached, for example, by a strap 103 to the wearer's head. In a second embodiment of the invention, one or more area light sources such as 101A and 101B may be used as shown in Figure 2. Figure 3 illustrates a third embodiment of the invention in which the emitter and lightguide 101 might take up substantially the entire area of the light mask to maximize the light output or to reduce the luminance required from the source.

There are several important benefits with this approach. First, heat dissipation becomes negligible and no area of the mask feels warm to the wearer. Second, the invention enables the use of low intensity light sources or to operate light sources at extremely low luminance. The mask can utilize a number of light sources, for example OLED (organic light emitting diodes), electroluminescent films, chemiluminescent layers, phosphorescent layers or even bioluminescent materials.

In one embodiment, OLED is used as the light source. The OLED source is ideally 2-1000 times larger than the emissive area required for the eyes. For example, the emissive OLED face can be 20x5cm while the area illuminating the eyes can be 1x1cm for each eye. The OLED area can take up substantially the whole area of the eye mask. Correspondingly, the OLED device is run at lower luminance. The device can be operated at reduced voltages and current density, which increases the lifetime of the OLED source. Reduced voltage operation is highly desirable for portable devices. In many OLED sources the power efficiency is improved at low luminance. Thus the invention also enables such devices to be run in a regime where they are more efficient.

In another embodiment long decay phosphorescent materials are used as the light source. The phosphorescent source is ideally 2-1000 times larger than the emissive area required for the eyes. For example, the emissive phosphorescent layer can be 20x5cm while the area illuminating the eyes can be 1x1cm for each eye. The phosphorescent layer can take up substantially the whole area of the eye mask. In turn the light output of the phosphorescent layer is amplified to higher intensity by the light guide. The invention therefore enables very low intensity afterglow materials to be used, yet provides sufficient light levels for the treatment of the eye.

In yet another embodiment, chemiluminescent or bioluminescent materials are used as the light source. The chemiluminescent source is ideally 2-1000 times larger than the emissive area required for the eyes. For example, the emissive chemiluminescent surface can be 20x5cm while the area illuminating the eyes can be 1x1cm for each eye. The chemiluminescent layer can take up substantially the whole area of the eye mask. In turn the light output of the chemiluminescent layer is amplified to higher intensity by the light guide. The invention therefore enables very low intensity chemiluminescent materials to be used, yet provides sufficient light levels for the treatment of the eye.

The light guide used in the mask can propagate light towards the eye using adjacent layers of low optical index materials or reflective layers. The light guide in the mask may optionally comprise at least one luminescent dye to effectively capture the light emitted by the area source. The dye may be used to define the spectra of the light emitted into the eye.

In order to compensate for variations of light intensity with time, the mask may comprise one or more photochromic layers. For example, phosphorescent or chemiluminescent sources exhibit decaying luminance with time. By including a photochromic member, it is possible to reduce the initial brightness and make the output illumination more even with time. As the luminance of the area source decays the photochromic layer becomes gradually more transparent, effectively providing a simple compensation mechanism.

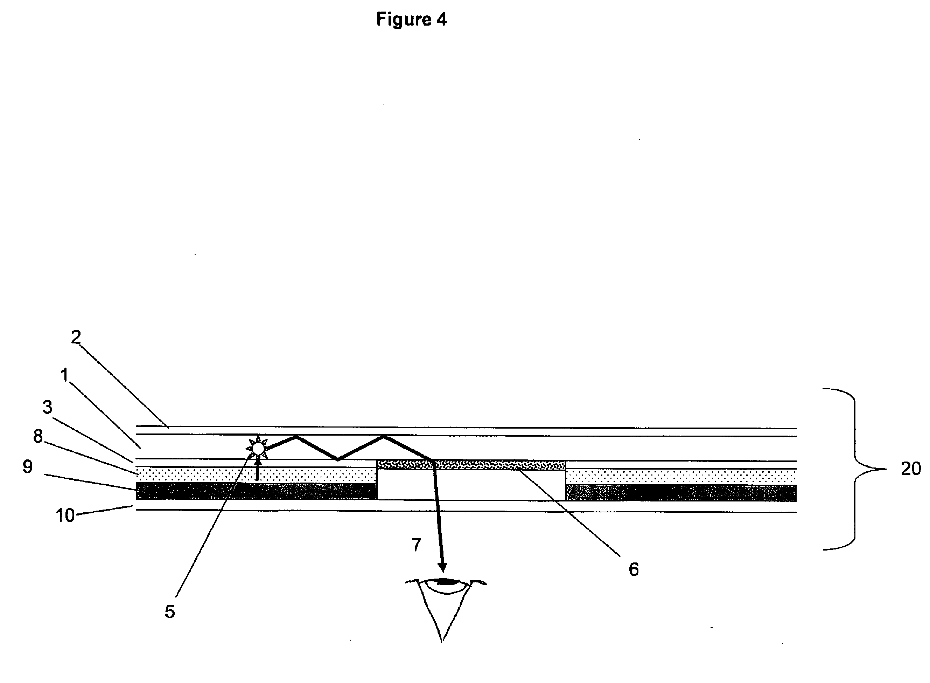

Figure 4 is a crossection view of an area of the mask 20 with an area emitter with the eye region 6 emitting light into the eyes or onto the eyelids of the patient. The device comprises a lightguide 1 optionally doped with a luminescent dye. Layers 2 and 3 are low index materials designed to ensure total internal reflection of the light entering layer 1. An emissive layer 8 is provided, with an area substantially larger than the eye region 6. Light emitted by emissive layer 8 is absorbed by the dye 5 and is reemitted into layer 1. The path of light 7 is then guided via internal reflection to the eye region 6. The mask may further comprise optionally a reflective layer 9 and a protective layer 10. The eye region 6 comprises a light outcoupling feature such as embossed or moulded features. This region may also contain pigment particles to scatter light for efficient outcoupling. Any suitable outcoupling technique can be used.

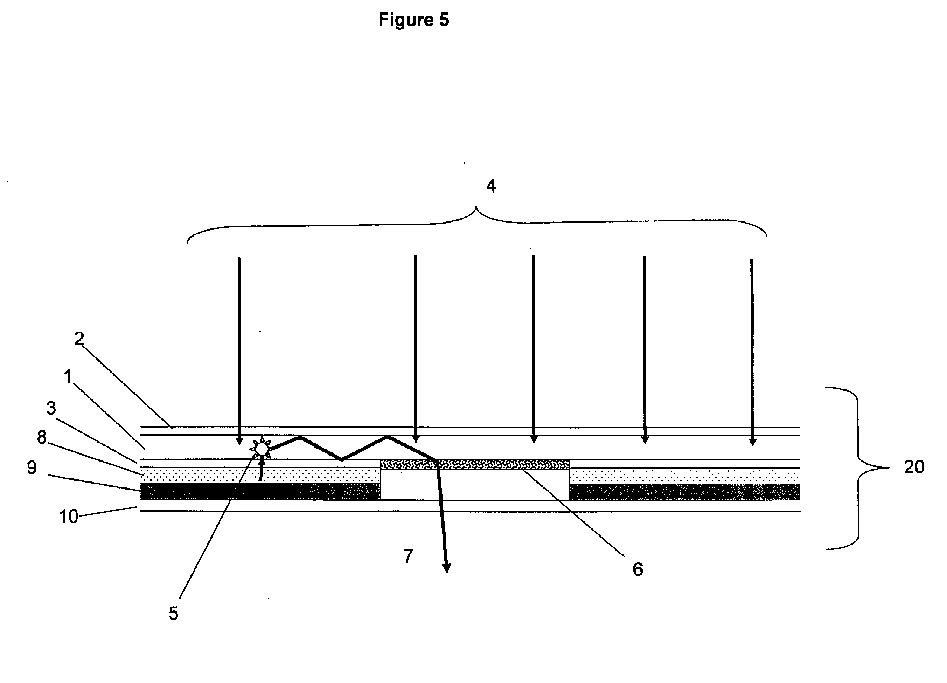

Figure 5 illustrates a technique for charging the mask when the emissive layer 8 comprises long afterglow phosphorescent materials. The layer 8 can be charged by external incident light 4, which can be ambient light or any suitable light source. In this embodiment the device might be charged by a dedicated light charger unit. The charger may be an enclosure containing one or more illuminating sources such as LEDs or incandescent bulbs.

The charger may also be in the form of an illuminator sheet with which the mask is brought into contact.

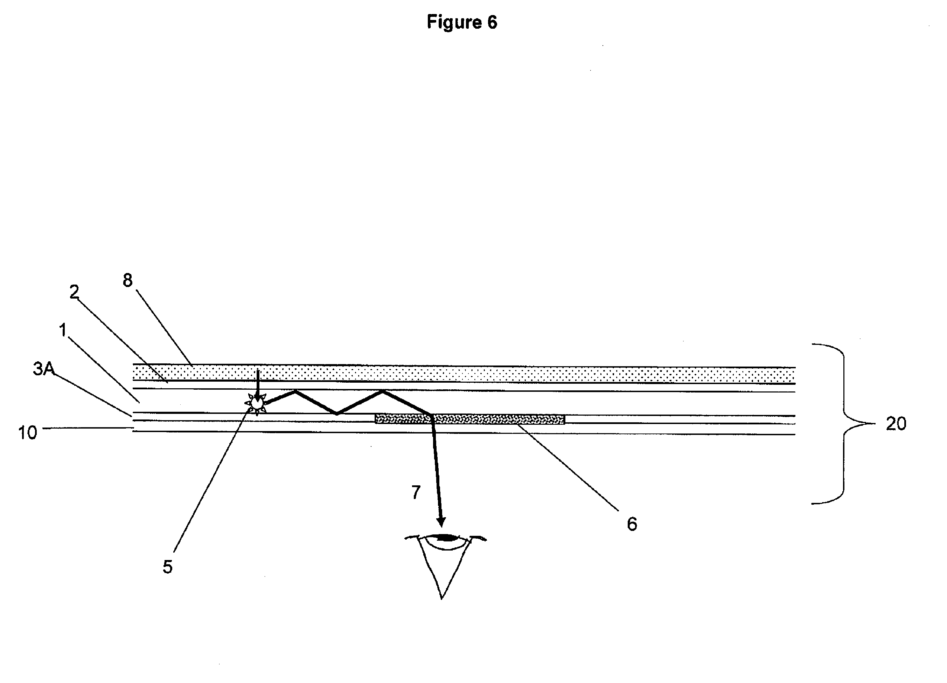

Figure 6 is a crossection view of a mask 20 with an area emitter 8 and the eye region 6 emitting light into the eyes or onto the eyelids of the patient. The device comprises a lightguide 1 optionally doped with a luminescent dye. Layers 2 and 3A are low index materials designed to ensure total internal reflection of the light entering layer 1. Layer 3A may optionally be a reflective layer such as metal. The light emitting layer 8 has an area substantially larger than the eye region 6. Light emitted by 8 is absorbed by the dye 5 and is reemitted into layer 1. The path of light 7 is then guided via internal reflection to the eye region 6. The mask may further comprise a protective layer 10.

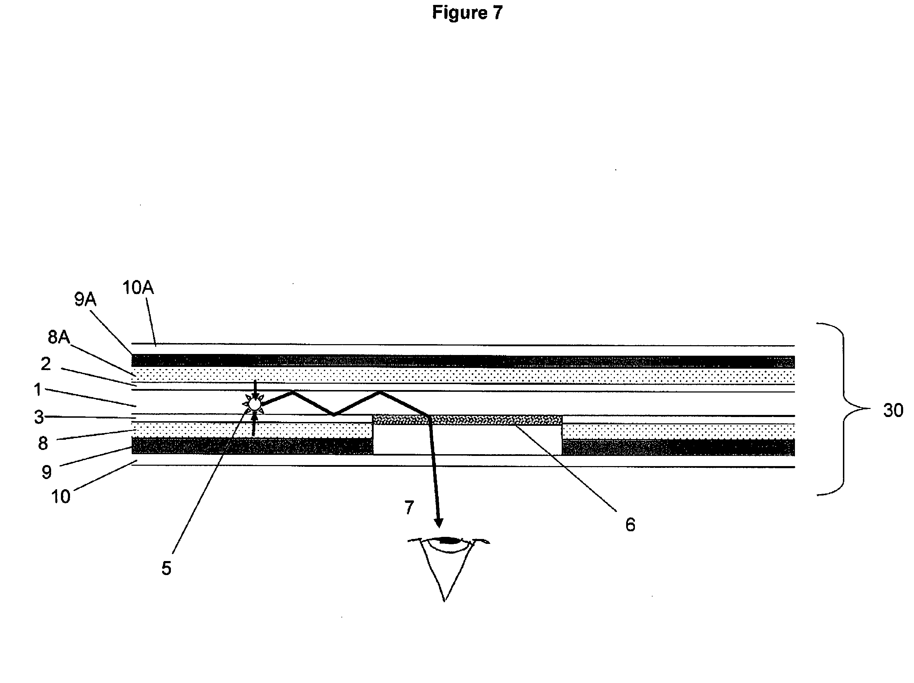

Figure 7 is a crossection view of a mask 30 with area emitters 8 and 8A on both sides of the lightguide 1 in order to further increase the area of the source. An optional reflective layer 9A is provided to prevent escape of the light from the surface of the mask. In this embodiment the total light output can be further increased or the luminance of the source reduced. The eye region 6 emits light into the eyes or onto the eyelids of the patient. The device comprises a lightguide 1 optionally doped with a luminescent dye. Layers 2 and 3A are low index materials designed to ensure total internal reflection of the light entering layer 1. The light emitting layers 8 and 8A together have an area substantially larger than the eye region 6. Light emitted by light emitting layers 8 and 8A is absorbed by the dye 5 and is reemitted into layer 1. The path of light 7 is then guided via internal reflection to the eye region 6. The mask may further comprise protective layers 10 and 10A.

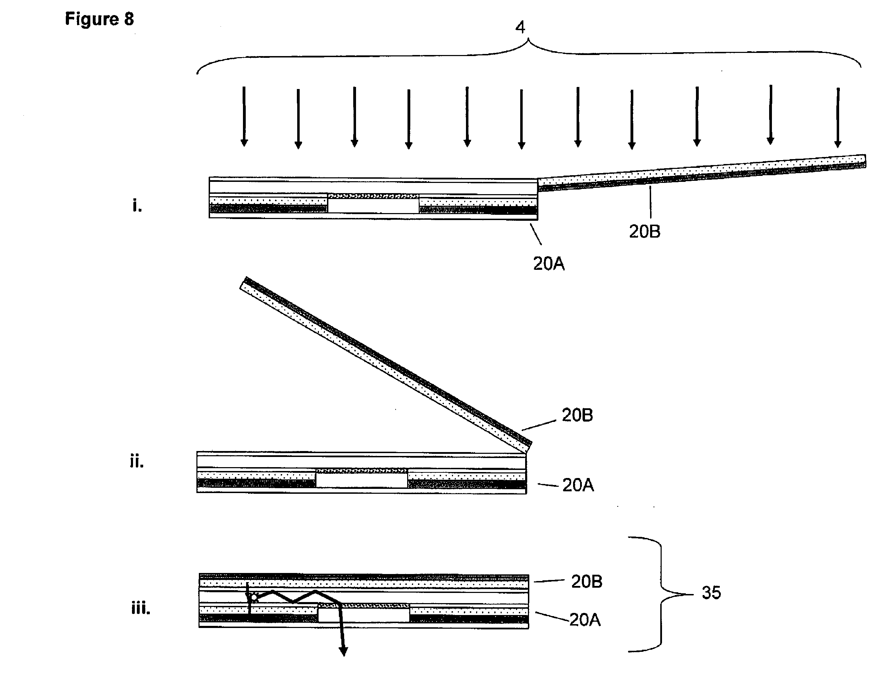

Figure 8 illustrates a mask having a foldable structure with two sets of area emitters comprising segments 20A and 20B. In one embodiment, the emitters comprise long afterglow phosphorescent layers in each segment. This mask can be conveniently charged in the open state as shown in Fig 8(i). External light 4 from the ambient or from a light charger unit excites the phosphorescent material causing it to glow following excitation. The mask can be folded as shown in Fig. 8(ii). The mask 35 is shown in its closed state in Fig.8(iii). Light irradiated from either side is captured in the lightguide and propagated toward the eye via internal reflection. In this embodiment the emissive area is significantly larger than the area to illuminate the eyes. For example, the total emissive area can be as large as 400 cm², while each of the areas illuminating the eye can be 1 cm².

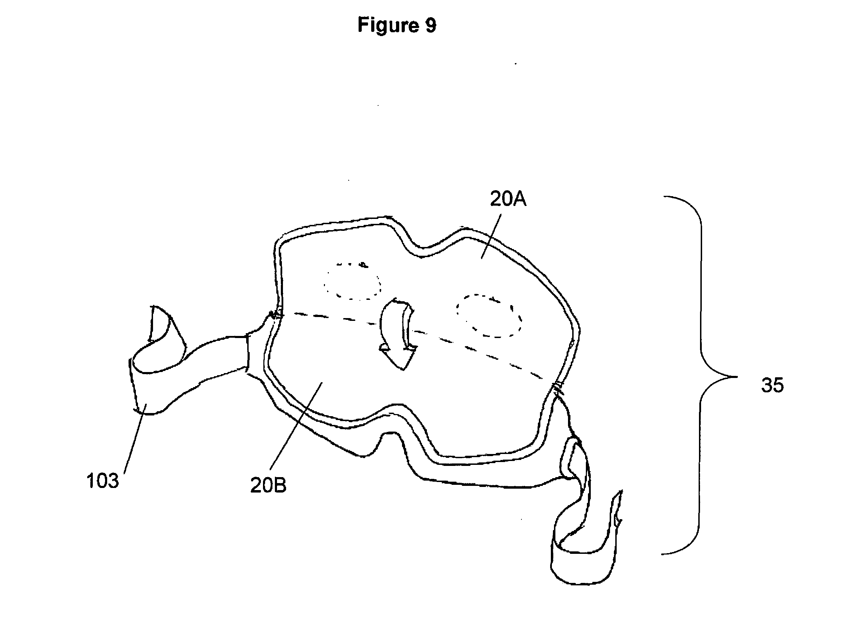

Fig.9 further illustrates a foldable eyemask 35 with two parts 20A and 20B having absorbing/emitting layers. Preferably the part 20A shining light on the eye is folded inwards to secure it to the head when strapped.

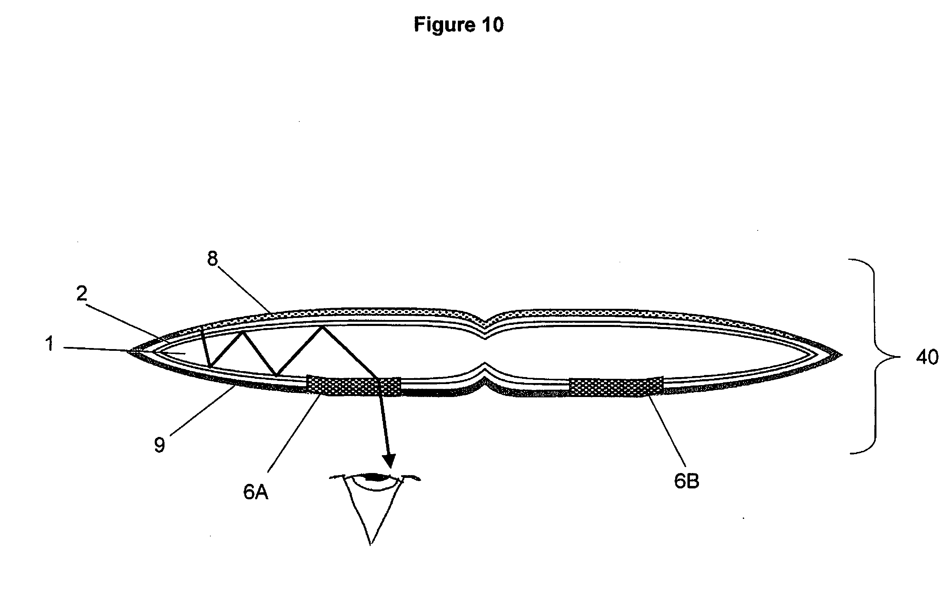

Fig.10 illustrates a preferred embodiment of the mask 40 in which the lightguide 1 is tapered or narrowed towards the edge of the mask. This reduces the number of reflections for the light emitted at the edge due to the gradually increasing angle of reflection from the low index layer 2 and/or reflective layer 9. The edge of the lightguide might also be rounded to reduce outcoupling and loss of light at the edge. In this embodiment light emitted from the area source 8 is coupled into the lightguide material 1. The lightguide may optionally be doped with a dye. Outcoupling is effected via regions 6A and 6B.

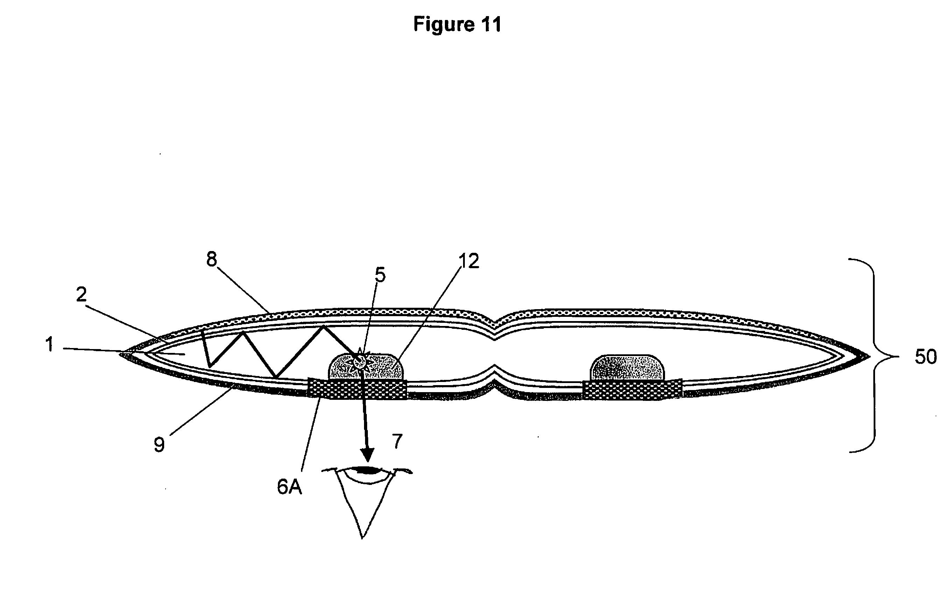

Figure 11 is further embodiment where an additional region 12 is disposed within the lightguide. Region 12 is doped with a dye 5 to effect conversion or correction of the emitted wavelength of the light 7.

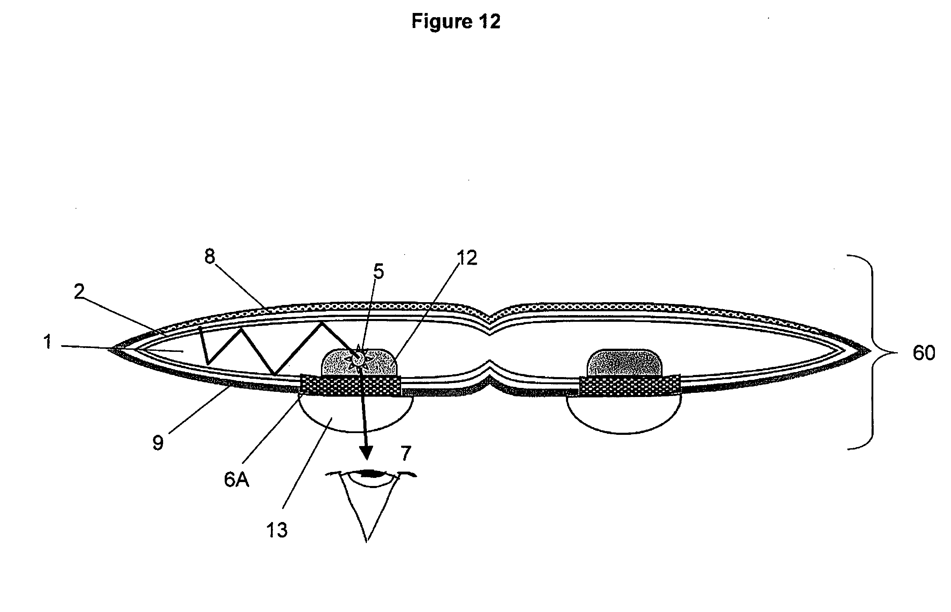

Figure 12 illustrates another embodiment, in which the eyemask comprises a further optical element 13 to couple light in close proximity to the eye or eyelid. Element 13 may be a polymer optionally shaped into a lens. This portion may also comprise a gel material to make soft contact with the eyelids. The gel may have additional functionality of cooling, for example, by placing it in a refrigerator prior to use.

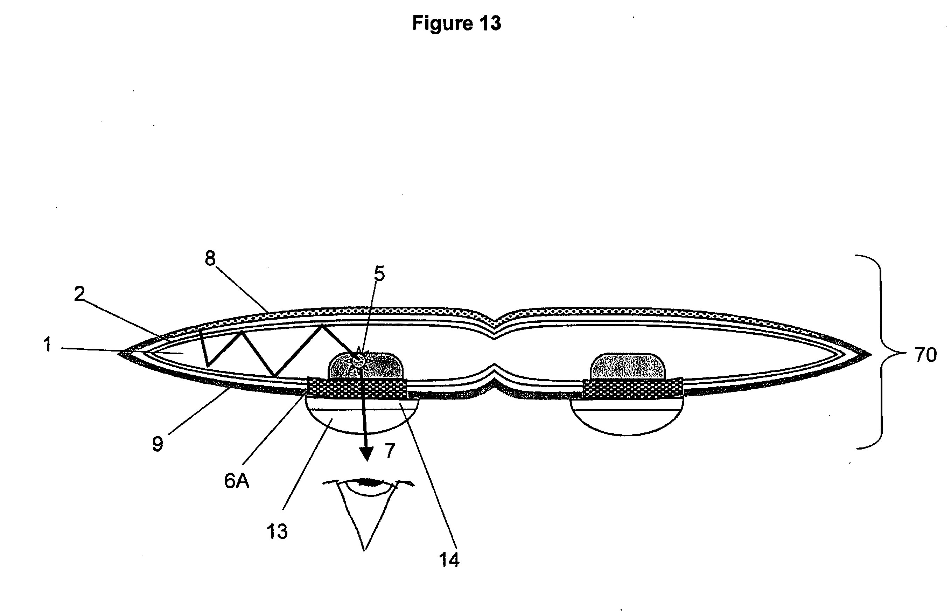

Figure 13. illustrates a further embodiment with a light controlling layer 14. This layer may comprise a photochromic material, which attenuates the light at high intensities while staying substantially transparent at low intensities. Such an element is an effective method to control light intensity variations from the source and is especially useful when the light source is phosphorescent or chemiluminescent.

### Sources:

The invention may utilize any area source, for example OLED, bioluminescent, phosphorescent coatings, e.g. luminophore, or chemiluminescent emitters. The mask might comprise one or more area sources, for example two sources on each side of the mask with the lightguide positioned in the middle. The two sides may form a foldable structure. To benefit from sources of low luminance the area of the source is at least twice the area of the outcoupling region provided to illuminate the eyes. Preferably the source is between 2 to 1000 times than the outcoupling region. More preferably, the source is between 4 to 200 times larger than the outcoupling region. Even more preferably the source is between 10 to 100 times the size of the area illuminating the eye. The area illuminating the eyes or eyelids is preferably between 1 mm² to 20 cm².More preferably, the area illuminating the eyes or eyelids is between 0.1 cm² to 10 cm². Even more preferably, the area illuminating the eyes or eyelids is between 1 cm² to 5 cm². The source should be structured in a manner that improves light coupling into the waveguide or on to the wavelength conversion materials. Such structuring may include the formation of 2D or 3D photonic crystals to direct the light emission or larger scale structures formed by, for example, deposition onto non-planar surfaces such as microprisms, or moulding into non-planar shapes.

### OLED sources

OLED sources might include luminescent or phosphorescent emitters. OLED films can be manufactured by any suitable technique, for example evaporation, solution coating techniques or printing. The source might comprise small molecule or polymeric materials. The emission colour can be defined by the choice of OLED materials. The OLED source comprises at least one emitting surface coupling to the lightguide. Optionally inorganic thin film luminescent or electroluminescent layers might be used. These may include inorganic quantum dots or inorganic luminescent or transport materials instead or in addition to OLED materials. The OLED source can be provided on a rigid substrate such as glass. Ideally the OLED source is provided on a flexible substrate such as polyester (PET) or polyethylene naphthalene (PEN). Any suitable polymeric substrate and barrier material can be used. Since the invention enables running the OLED at lower luminance, less perfect encapsulation is acceptable. It is estimated that in an optimized mask configuration, the luminance of the OLED source can be reduced by a factor of 2-100.

When OLED area sources are used the mask comprises a power source such as a battery. Low voltage (<10V) operation is preferred for safety reasons as well as for reducing the size and complexity of the power source. Ideally the power source is less than 6 V, more preferably less than 3 V. Importantly, the invention enables running OLED at low voltages and current densities.

### Light accumulation sources

In one embodiment the area light source comprises phosphorescent or luminescent materials that exhibit long glow after excitation. For example, following a light exposure they glow for minutes or hours. Such materials make it possible to provide battery free phototherapy devices with the capability to recharge the device for prolonged light emission. Materials with delayed phosphorescence excited by any electromagnetic radiation can be considered, for example infrared light or heat. Such materials can be formed into a film, sheet or coating to provide an area light source. The sheet or coating may comprise substantially the entire area of the mask, coupling light into the lightguide described in the present invention.

Materials particularly relevant to the invention are long decay luminophore materials where the re-emitted light is slowly released over a period of time after initial excitation. Suitable materials include, but are not limited to, those described in US patent 5424006 and US Patent 5686022. Suitable materials include those manufactured by Nemoto & Company, Tokyo and are available under the brand name LUMINOVA having the general formula MAl₂O₄, where M is one or more metals selected from strontium (Sr), calcium (Ca), barium (Ba), magnesium (Mg) activated by europium (Eu) and at least one co-activator selected from lanthanum (La), cerium (Ce), praseodymium (Pr), neodymium (Nd), samarium (Sm), gadolinium (Gd), dysprosium (Dy), holmium (Ho), erbium (Er), terbium (Tb), thulium (Tm), ytterbium (Yb), lutetium (Lu), tin (Sn), manganese (Mn) and bismuth (Bi). Chemical compositions of exemplary materials for use in the invention include, but are not limited to: SrAl₂O₄: Eu; SrAl₂O₄: Eu, Dy; SrAl₂O₄: Eu, Nd; Sr₄Al₁₄O₂₅:Eu; Sr_{0.5} Ca_{0.5} Al₂O₄: Eu , Dry; BaAl₂O₄: Eu, Nd; BaAl₂O₄: Eu, Sn; ZnS:Cu; ZnS:Cu, Co; ZnS:Mn; ZnS:Ag; BaMgAl₁₀O₁₇:Eu; BaMgAl₁₀O₁₇ : Mn, Eu; Sr₂P₂O₇ : Eu; CaWO₄; CaWO₄ : Pb; SrGa₂S₄ : Eu; CePO₄ : Tb; MgW04; Y₂O₃ : Eu; Y₃A1₅O₁₂ : Ce; (Ba₁-ₓSrₓ) ₅ (PO₄) ₃ (F, C1) :Eu; (Y_{1-x-y}GdₓLu_{y})₃ (Al_{1-y}Ga_{y})₅O₁₂:Ce; (Y₁-xGdx) ₂O₃:Bi, Eu; (Y₁₋ₓPₓ)₂O₄: Eu; YVO₄:Eu; Y₂O₂S : Eu.

### Chemiluminescent sources

Chemiluminescent sources may also be provided as the area light emitter. Such light sources exploit the light emission associated with the product of the chemical reaction between at least two reactants. A common example of such light source is the so-called glow stick where a fluorescent dye is excited by the decomposition of the product of the reaction between hydrogen peroxide and oxalate ester. Particularly attractive to the invention in the case of a single use phototherapy device is a system where one of the reactants is contained in a vessel which is broken by application of pressure (or folding) to allow the reactants to mix and initiate the chemical reaction responsible for light emission. The reactants may be included in a sheet comprising a gel. The sheet may take up substantially the entire area of the mask, coupling light into the lightguide described in the present application.

### Lightguides:

The lightguide may comprise substantially the whole area of the eye mask. Preferably the lightguide comprises between 5% to 100% of the mask area. More preferably the lightguide comprises between 10% to 95% of the mask area. The lightguide is the same size or larger than the light source. Suitable lightguide constructions include a polymer core with a high index material and surrounding low index polymer layer(s), which may extend to both sides of the lightguide. Where required, for example in the case where the refractive index of the light source (i.e. OLED source) is substantially higher than the low index layer or even the higher index core, an additional high index coupling layer such as narrow angle high gain GRIN diffusers (for example those offered by Microsharp Ltd) can be placed in between the source and waveguide. Where a wavelength conversion layer is used, an appropriate position would be between the low index cladding and high index core, in order to take advantage of Supercritical Angle Fluorescence (SAF) which allows the preferential injection of converted light into waveguided modes of the high index core. Appropriate materials for the waveguide should have a high degree of transparency in the spectral region of interest, and where required should be flexible and appropriate for the particular method of manufacture. Low index materials therefore may include MY-132 (MY Polymers Ltd), with refractive index 1.32. A high index core could include material such as polysiloxanes (WO/2003/011944) with refractive index of up to 1.581. This combination of materials would have a critical angle of 58 degrees, and SAF emission from the interface would couple up to approximately 70% of light directly into the waveguided modes.

These may include polystyrenes, poly(ethylene terephthalate), a transparent polyolefin, in particular a clarified polyolefin, for example clarified polypropylene, Poly(methyl methacrylate) (PMMA), transparent polyamide or polycarbonate and perfluorinated polymers such as polyperfluorobutenylvinylether, polysulphones,polyether sulphones and polyacrylates. PMMA and polycarbonate are two transparent thermoplastics of choice because of their ease of processing, their availability on the market, their high transparency in the visible range and refractive index in the visible range. Other materials which are recommended for use with fluorescent dyes include styrene-butadiene. For the low index cladding, polymers such as TEFLON FEP, PCTFE (polychlorotrifluoro-ethylene) or PVDF as well as UV curable low index polymers such as the OPTI CLAD (manufactured by Ovation Polymers) series, may be chosen, which have refractive indices as low as 1.33. Further examples of polymers can be found in "Encyclopedia of Polymer Science and Engineering", 2nd Edition, J Wiley and Sons and "Polymer Handbook", 4th ed.; John Wiley & Sons, 1999.The lightguides may be further surrounded by reflectors, as described in a later section in order to recycle non-waveguided light, both allowing light from the initial light source to be down converted on later passes and also recycle non-SAF emitted light. In general, structures constructed so that the majority of the light is waveguided by Total Internal Reflection (TIR) will have better device performance. Additionally the high index core of the lightguide may be doped with light accumulation or wavelength converting chemical. The ends of the lightguides may be squared and mirrored, or they may be tapered to a point or curved edge such as a parabola such that waveguided light may be turned and reflected towards the centre of the guide again with minimal reflections from metallic surfaces. The lightguide may also be a gel material within a polymer shell.

### Wavelength conversion materials

The lightguide may comprise wavelength conversion materials such as dyes. Such materials can aid coupling of light into the lightguide. Wavelength conversion is also an approach to adjust the wavelength emitted into the eyes. By using such materials it is possible to convert less effective wavelength light into an effective spectrum. Suitable materials or mixtures of materials are those which absorb light at a first wavelength and subsequently emit light at a second wavelength which is different from the first wavelength. The emitted light might be result of fluorescence and/or phosphorescence depending on the type of material or mixture of materials. In addition the nature of the material or mixture of materials and associated energy conversion mechanisms will dictate if the wavelength emitted is larger or smaller than that of the absorbed light. Light can be absorbed by one species and re-emitted by a second species following non-radiative energy transfer.

Suitable wavelength conversion materials may be of any type including but not limited to coumarins; perylenes; xanthenes; thioxanthenes; fluoresceins; rhodamines; azlactones; methines; oxazines; thiazines; phtalocyanines; stilbenes; distilbenes; distyrenes; azomethines; phenanthrenes; rubrene; quinacridones; naphtalimides; methines; pyrazolones; quinophthalones; perinones; anthraquinones. Materials particularly relevant to the invention are those which can be efficiently doped into light guide materials without compromising their optical function and which exhibit a high quantum yield. Of particular interest are fluorescent dyes manufactured by BASF and available under the brand name LUMOGEN F Dyes. Other commercial materials of interest include those available under the following brand names MACROLEX (Bayer), HOSTASOL (Clariant), THERMOPLAST (BASF), SOLVAPERM (Clariant), SANDOPLAST (Clariant), AMAPLAST (Color-Chem).

Quantum dot materials may also be used to adjust the emission characteristics of the mask. Suitable quantum dots materials include but are not limited to CdSe, CdTe, ZnS, ZnTe, ZnSe, CdS, HgS, HgSe, HgTe, CdTeSe, CdTeS, ZnSSe, ZnTeSe, ZnSTe, CdZnSe, CdZnTe, CdZnS, GaAs, GaP, GaSb, GaN, InN, InP, InAs, InSb, InGaP, InGaAs, InGaN, AlInGaN, AIInGaP, AlInGaAs, Si, Ge.

The above wavelength conversion materials may also be used as part of the area light source or the outcoupling region.

### Outcoupling methods

The mask comprises at least one outcoupling region as an exit window to shine light onto the eye. Outcoupling from this region may be achieved through the use of scattering, embedded nanoparticles and/or quantum dots which may be doped or undoped, of various shapes and orientations such as rods, triangles or spheres, optical interference structures such as diffraction gratings or holographic structures, photonic crystals which may be either 2D or 3D, macroscopic shaped structures such as, but not limited to, micropyramids and microprisms, microlenses, lens arrays, GRIN structures, fiber optics for either scattering or redirection of light through waveguiding or other coupling methods through variation of refractive index. The outcoupling region may either be directly on the side of the escape window area forming part of the window, it may be an external component attached to the window, or it may be on the opposite side of the waveguide to the window (i.e. diffractive components) Additionally bulk components such as scattering particles, nanoparticles and lumophores may be present in the waveguide near the window for outcoupling, or they may form part of external components. Concave or convex outcoupling schemes may be employed.

### Reflectors

Reflectors will have two primary purposes. The first is to ensure that light that is not wavelength converted and will be substantially reflected through the wavelength conversion region so that a significant proportion of light will be converted on later passes as opposed to being absorbed and lost. The second is to provide supplementary waveguiding to light intended for emission that has not been injected into supercritical waveguided modes in the waveguide core. In the case of the SAF layer above, this would make up around 30% of the light. The reflectors may consist of periodic or aperiodic dielectric multilayers which may be either isotropic or anisotropic in nature, with the particular arrangements of refractive indices, thicknesses and index ellipsoid orientations chosen to optimise reflections of light which is not captured within the waveguide to either ensure its passage to the outcoupling region or to increase the chances of absorption and re-emission at the wavelength conversion layers. Such materials may either be custom arrangements of layers which may be extruded or laminated onto the core or commercially available films. The choice of materials may also be chosen to allow their dual use as barrier layers to lengthen the lifetime of the devices. In the event that no practical arrangements can be found, it would also be possible to used metallic or metallised layers as a reflector. However for large numbers of reflections, the efficiencies of metallic layers are significantly poorer.

### Optical modulation layer

The mask may optionally comprise one or more optically variable layers for modulation of the light coupling. This may for example be a photochromic light absorber (such as silver chloride) or may change refractive index such that index matching between layers (3) or (5) and (9) increases and decreases according to the desired conditions. Such layers that change properties based on conditions including but not limited to, pH and chemicals (chemochromic), humidity (hydrochromic), temperature (thermochromic) and pressure may also be used. The optically variable layer may be between the light source and waveguide, in the waveguide outcoupling area, or in the additional pad (13). The layer may consist of pure photochromic chemical, a doped material such as polymer, or be dispersed in a solution. The range of suitable materials is large and includes inorganic materials such as zinc halides and silver chloride, as well as organic materials such as spirooxazines which have fast switching in gels and spiropyrans which can be cross linked with other molecules. Such organic molecules as these may be tuned to specific wavelength switching requirements.

### Shape, form of implementation

The eyemask is ideally flexible or conformal. The mask may be provided for both eyes or for one eye or as a patch. The form factor and bend angles of the waveguide should remain small with respect to the thickness of the waveguide in order to ensure an acceptable level of total internal reflection is maintained. The lightguide may comprise a soft material such as a gel. The gel may provide an additional cooling function to soothe the eyes. The external surface of the mask may be covered with a fabric material or other soft layer or layers such as a foam. These layers can have the function to provide vapour transmission.

### Method for manufacturing

The eyemask can be manufactured by a variety of techniques. The lightguide is preferably manufactured by moulding, lamination, stretching, forming of the lightguide as well as emissive components. The outcoupling features can be formed in a single moulding step when forming the mask or defined in a second step by embossing or coating further layers. The area light source such as OLED or phosphorescent source may be laminated or attached to the lightguide using an adhesive. The source may also be directly coated onto the lightguide by solution coating technique such as screen-, flexo-, gravure-, or inkjet printing, spray coating or dip coating. The source may also be deposited on the lightguide by evaporation.

### Examples

### Example 1

### Retinopathy device

In patent application GB2410903A, Arden describes an eye mask device using inorganic LED light sources to illuminate the eyes of patients suffering from retinal diseases during their sleep. Because the treatment is carried out during sleep a phototherapy device with minimum discomfort for the user is advantageous. Because of the low light intensity required and the long illumination time available a passive phototherapy device using luminophore for slow release of light is particularly suitable. This example describes such device.

A light guide sheet element (less than 2 mm thick in thickness) could be moulded in the shape of an eye mask as to cover the eye of a patient as shown in Figure 1. The element is formed by injection moulding silicone resin containing 0.1% of Lumogen F Green 850 (BASF). The light guide is coated on both sides with a 5 microns thick lower index polymer such as polychlorotrifluoro-ethylene.

A blue light emitting OLED is formed on top of the lightguide using 4,4'-bis(2,2'diphenilvinyl)-1,1'-biphenyl (DPVBi) as the emitter, for example by using a device structure described by Othman et al in Proc. IEEE of ICSE, 2004. The blue emission centered at 483 nm is then absorbed by the lightguide and transmitted to the emitting surface.

On the opposite side of the OLED, two light delivery areas are defined to coincide with the eye of the patient so that the light emitted by the phototherapy device can illuminate the eye lids of the patient. These areas assist outcoupling by surface structuring to the attachment of a soft sac containing mineral oil or silicone gel of an equal or higher refractive index to the core. This allows light to be coupled directly from the lightguide core into a non-waveguiding structure directly over the eye, and allowing light into the eye. The use of a gel further assists comfort and coupling between the lightguide mask and eye. A large proportion of the light emitted by the OLED enters the lightguide, where it is absorbed by the fluorescent die and re-emitted as light with peak wavelength around 500 nm. The 500 nm light is emitted through the emitting surface is adequate for absorption rod cells.

It will be appreciated by persons skilled in the art that the above embodiments have been described by way of example only and not in any limitative sense, and that various alterations and modifications are possible without departure from the scope of the invention as defined by the appended claims.

## Claims

1. An apparatus (100, 20) for directing electromagnetic radiation into a respective pupil of at least one eye of a user, the apparatus comprising:
first radiation emitting means comprising at least one radiation emitting layer (8) adapted to emit electromagnetic radiation;
second radiation emitting means (6) adapted to direct electromagnetic radiation into at least one eye and/or onto at least one eyelid of a user; and
radiation guide means (1, 2, 3) adapted to receive radiation emitted by said first radiation emitting means and to direct at least part of said radiation to said second radiation emitting means, wherein a total surface area of said first emitting radiation means from which radiation is emitted is larger than a total cross sectional area of a beam of radiation directed to the or each pupil of the user by the second radiation emitting means (6), in a direction transverse to an optical axis of said beam.

2. An apparatus according to claim 1, wherein at least one said radiation emitting layer is adapted to emit radiation in response to irradiation of said layer.

3. An apparatus according to claim 1 or 2, wherein at least one said radiation emitting layer comprises at least one organic light emitting diode (OLED).

4. An apparatus according to any one of the preceding claims, wherein the radiation guide means comprises a radiation transmitting portion including at least one first layer having a respective first refractive index and at least one second layer having a respective second refractive index, wherein said first refractive index of at least one said first layer is higher than the respective second refractive index of at least one second layer in contact with said first layer.

5. An apparatus according to any one of the preceding claims, wherein the radiation guide means includes at least one luminescent material.

6. An apparatus according to claim 5, wherein the degree of luminescence of at least one said luminescent material varies with time.

7. An apparatus according to claim 5 or 6, wherein the luminescent material is concentrated in a region adjacent said second radiation emitting means.

8. An apparatus according to any one of the preceding claims, wherein the radiation guide means further comprises at least one reflective layer (9).

9. An apparatus according to any one of the preceding claims, wherein the radiation guide means may comprises a radiation transmitting portion having tapered thickness adjacent at least one edge portion thereof.

10. An apparatus according to any one of the preceding claims, wherein the radiation guide means comprises a radiation transmitting portion, wherein the radiation transmitting portion is rounded adjacent at least one edge portion thereof.

11. An apparatus according to any one of the preceding claims, wherein the second radiation emitting means is adapted to conform to a shape of at least one eyelid of the user.

12. An apparatus according to any one of the preceding claims, wherein the second radiation emitting means includes at least one coolable material.

13. An apparatus according to any one of the preceding claims, wherein the second radiation emitting means includes at least one material having a transmissivity to radiation varying with radiation intensity.

14. An apparatus according to any one of the preceding claims, wherein at least one said radiation emitting layer is adapted to be moved between a respective first position, in which said layer is adapted to direct radiation into said radiation guide means, and a respective second position, in which said layer is adapted to be exposed to exciting radiation.

15. An apparatus according to claim 14, wherein at least one said radiation emitting layer is adapted to be moved between respective first and second positions thereof by folding.

## Patentansprüche

1. Vorrichtung (100, 20) zum Leiten von elektromagnetischer Strahlung in eine jeweilige Pupille von wenigstens einem Auge eines Benutzers, wobei die Vorrichtung Folgendes umfasst:
erste Strahlungsemissionsmittel mit wenigstens einer Strahlungsemissionsschicht (8) zum Emittieren von elektromagnetischer Strahlung;
zweite Strahlungsemissionsmittel (6) zum Leiten von elektromagnetischer Strahlung in wenigstens ein Auge und/oder auf wenigstens ein Augenlid eines Benutzers; und
Strahlungsleitmittel (1, 2, 3) zum Empfangen von von dem genannten ersten Strahlungsemissionsmittel emittierter Strahlung und zum Leiten wenigstens eines Teils der genannten Strahlung auf das genannte zweite Strahlungsemissionsmittel, wobei eine Gesamtfläche des genannten ersten Strahlungsemissionsmittels, von dem Strahlung emittiert wird, größer ist als eine Gesamtquerschnittsfläche eines Strahls von Strahlung, die von dem zweiten Strahlungsemissionsmittel (6) auf die oder jede Pupille des Benutzers in einer Richtung transversal zu einer optischen Achse des genannten Strahls emittiert wird.

2. Vorrichtung nach Anspruch 1, wobei wenigstens eine genannte Strahlungsemissionsschicht so ausgelegt ist, dass sie Strahlung als Reaktion auf die Bestrahlung der genannten Schicht emittiert.

3. Vorrichtung nach Anspruch 1 oder 2, wobei wenigstens eine genannte Strahlungsemissionsschicht wenigstens eine organische Leuchtdiode (OLED) umfasst.

4. Vorrichtung nach einem der vorherigen Ansprüche, wobei das Strahlungsleitmittel einen Strahlungsdurchlassteil mit wenigstens einer ersten Schicht mit einem jeweiligen ersten Brechungsindex und wenigstens einer zweiten Schicht mit einem jeweiligen zweiten Brechungsindex umfasst, wobei der genannte erste Brechungsindex von wenigstens einer genannten ersten Schicht höher ist als der jeweilige zweite Brechungsindex von wenigstens einer zweiten Schicht in Kontakt mit der genannten ersten Schicht.

5. Vorrichtung nach einem der vorherigen Ansprüche, wobei das Strahlungsleitmittel wenigstens ein lumineszentes Material beinhaltet.

6. Vorrichtung nach Anspruch 5, wobei der Grad an Lumineszenz von wenigstens einem genannten lumineszenten Material im Laufe der Zeit variiert.

7. Vorrichtung nach Anspruch 5 oder 6, wobei das lumineszente Material in einer Region neben dem genannten zweiten Strahlungemissionssmittel konzentriert ist.

8. Vorrichtung nach einem der vorherigen Ansprüche, wobei das Strahlungsleitmittel ferner wenigstens eine reflektierende Schicht (9) umfasst.

9. Vorrichtung nach einem der vorherigen Ansprüche, wobei das Strahlungsleitmittel einen Strahlungsdurchlassteil von sich verjüngender Dicke neben wenigstens einem Randabschnitt davon umfasst.

10. Vorrichtung nach einem der vorherigen Ansprüche, wobei das Strahlungsleitmittel einen Strahlungsdurchlassteil umfasst, wobei der Strahlungsdurchlassteil neben wenigstens einem Randabschnitt davon gerundet ist.

11. Vorrichtung nach einem der vorherigen Ansprüche, wobei das zweite Strahlungsemissionsmittel so ausgelegt ist, dass es einer Form von wenigstens einem Augenlid des Benutzers entspricht.

12. Vorrichtung nach einem der vorherigen Ansprüche, wobei das zweite Strahlungsemissionsmittel wenigstens ein kühlbares Material beinhaltet.

13. Vorrichtung nach einem der vorherigen Ansprüche, wobei das zweite Strahlungsemissionsmittel wenigstens ein Material mit einer Strahlungsdurchlässigkeit beinhaltet, die mit der Strahlungsintensität variiert.

14. Vorrichtung nach einem der vorherigen Ansprüche, wobei wenigstens eine genannte Strahlungsemissionsschicht so ausgelegt ist, dass sie zwischen einer jeweiligen ersten Position, in der die genannte Schicht Strahlung auf das genannte Strahlungsleitmittel leitet, und einer jeweiligen zweiten Position bewegt wird, in der die genannte Schicht Erregungsstrahlung ausgesetzt wird.

15. Vorrichtung nach Anspruch 14, wobei wenigstens eine genannte Strahlungsemissionsschicht so ausgelegt ist, dass sie zwischen jeweiligen ersten und zweiten Positionen davon durch Falten bewegt wird.

## Revendications

1. Appareil (100, 20) pour diriger un rayonnement électromagnétique dans une pupille respective d'au moins un oeil d'un utilisateur, cet appareil comprenant :
un premier moyen émetteur de rayonnement comprenant au moins une couche émettrice de rayonnement (8) adaptée de façon à émettre un rayonnement électromagnétique ;
un deuxième moyen émetteur de rayonnement (6) adapté de façon à diriger un rayonnement électromagnétique dans au moins un exil et/ou sur au moins une paupière d'un utilisateur ; et
des moyens de guidage de rayonnement (1, 2, 3) adaptés de façon à recevoir le rayonnement émis par ledit premier moyen émetteur de rayonnement et à diriger au moins une partie dudit rayonnement sur ledit deuxième moyen émetteur de rayonnement, une superficie totale dudit premier moyen émetteur de rayonnement duquel le rayonnement est émis étant plus grande qu'une superficie en coupe totale d'un faisceau de rayonnement dirigé sur la ou chaque pupille de l'utilisateur par le deuxième moyen émetteur de rayonnement (6), dans une direction transversale à un axe optique dudit faisceau.

2. Appareil selon la revendication 1, dans lequel au moins une dite couche émettrice de rayonnement est adaptée de façon à émettre un rayonnement en réponse à l'irradiation de ladite couche.

3. Appareil selon la revendication 1 ou 2, dans lequel au moins une dite couche émettrice de rayonnement comprend au moins une diode électroluminescente organique (DELO).

4. Appareil selon l'une quelconque des revendications précédentes, dans lequel les moyens de guidage de rayonnement comprennent une partie de transmission de rayonnement comprenant au moins une première couche ayant un premier indice de réfraction respectif et au moins une deuxième couche ayant un deuxième indice de réfraction respectif, ledit premier indice de réfraction d'au moins une dite première couche étant plus élevé que ledit deuxième indice de réfraction respectif d'au moins une deuxième couche en contact avec ladite première couche.

5. Appareil selon l'une quelconque des revendications précédentes, dans lequel le moyen de guidage de rayonnement comprend au moins un matériau luminescent.

6. Appareil selon la revendication 5, dans lequel le degré de luminescence d'au moins un dit matériau luminescent varie avec le temps.

7. Appareil selon la revendication 5 ou 6, dans lequel le matériau luminescent est concentré dans une région adjacente audit deuxième moyen émetteur de rayonnement.

8. Appareil selon l'une quelconque des revendications précédentes, dans lequel le moyen de guidage de rayonnement comprend en outre au moins une couche réfléchissante (9).

9. Appareil selon l'une quelconque des revendications précédentes, dans lequel le moyen de guidage de rayonnement peut comporter une partie de transmission de rayonnement ayant une épaisseur diminuée adjacente à au moins une partie de bord de celle-ci.

10. Appareil selon l'une quelconque des revendications précédentes, dans lequel le moyen de guidage de rayonnement comporte une partie de transmission de rayonnement, cette partie de transmission de rayonnement étant arrondie dans une position adjacente à au moins une partie de bord de celle-ci.

11. Appareil selon l'une quelconque des revendications précédentes, dans lequel le deuxième moyen d'émission de rayonnement est adapté de façon à se conformer à une forme d'au moins une paupière de l'utilisateur.

12. Appareil selon l'une quelconque des revendications précédentes, dans lequel le deuxième moyen émetteur de rayonnement comprend au moins un matériau pouvant être refroidi.

13. Appareil selon l'une quelconque des revendications précédentes, dans lequel le deuxième moyen émetteur de rayonnement comprend au moins un matériau ayant une transmissivité au rayonnement variant avec l'intensité du rayonnement,

14. Appareil selon l'une quelconque des revendications précédentes, dans lequel au moins une dite couche émettrice de rayonnement est adaptée de façon à être bougée entre une première position respective, dans laquelle ladite couche est adaptée de façon à diriger le rayonnement dans lesdits moyens de guidage de rayonnement, et une deuxième position respective, dans laquelle ladite couche est adaptée de façon à être exposée à un rayonnement d'excitation.

15. Appareil selon la revendication 14, dans lequel au moins une dite couche émettrice de rayonnement est adaptée de façon à être bougée entre une première et une deuxième position respective de celle-ci par pliage.
